(19) Europäisches Patentamt
European Patent Office
Office européen des brevets

(11) Veröffentlichungsnummer : **0 008 079**
**B1**

# (12) EUROPÄISCHE PATENTSCHRIFT

(45) Veröffentlichungstag der Patentschrift : **17.03.82**

(51) Int. Cl.³ : **C 07 D213/74**, A 61 K 7/13

(21) Anmeldenummer : **79102772.5**

(22) Anmeldetag : **02.08.79**

## (54) Neue Bis-Aminopyridine, deren Herstellung und Verwendung als Komponenten in Oxidationshaarfarben sowie diese enthaltende Haarfärbemittel.

(30) Priorität : **07.08.78 DE 2834605**

(43) Veröffentlichungstag der Anmeldung : **20.02.80 (Patentblatt 80/04)**

(45) Bekanntmachung des Hinweises auf die Patenterteilung : **17.03.82 Patentblatt 82/11**

(84) Benannte Vertragsstaaten : **BE CH FR GB**

(56) Entgegenhaltungen : **US - A - 3 306 903**

(73) Patentinhaber : **Henkel Kommanditgesellschaft auf Aktien**
**-Patentabteilung- Postfach 1100 Henkelstrasse 67**
**D-4000 Düsseldorf 1 (DE)**

(72) Erfinder : **Rose, David, Dr.**
**Holbeinweg 7**
**D-4010 Hilden (DE)**
Erfinder : **Konrad, Günther, Dr.**
**Feuerbachweg 12**
**D-4010 Hilden (DE)**
Erfinder : **Lieske, Edgar**
**Hunsrückenstrasse 40**
**D-4000 Düsseldorf (DE)**

**EP 0 008 079 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

« Neue Bis-Aminopyridine, deren Herstellung und Verwendung als Komponenten in Oxidationshaarfarben sowie diese enthaltende Haarfärbemittel »

Gegenstand der Erfindung sind neue Bis-Aminopyridine der allgemeinen Formel

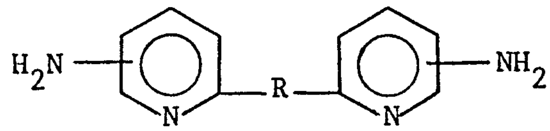

in der R die Gruppen $-HN-CH_2-NH-$, $-HN-CH_2-CH_2-NH-$ darstellen kann.

Weitere Gegenstände der Erfindung sind die Herstellung der neuen Bis-Aminopyridine, deren Verwendung als solcher oder in Gestalt ihrer Salze mit anorganischen oder organischen Säuren als Kuppler- und/oder Entwicklerkomponenten in Oxidationshaarfarben sowie Haarfärbemittel, die die neuen Bis-Aminopyridine bzw. deren Salze enthalten.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen wie p-Phenylendiaminderivate, Diaminopyridine, 4-Aminopyrazolon-derivate, heterocyclische Hydrazone eingesetzt. Als sogenannte Kupplerkomponenten werden m-Phenylendiaminderivate, Phenole, Naphthole, Resorcinderivate und Pyrazolone genannt.

Gute Oxidationshaarfarbstoffkomponenten müssen in erster Linie folgende Voraussetzungen erfüllen :

Sie müssen bei der oxidativen Kupplung mit den jeweiligen Entwickler- bzw. Kupplerkomponenten die gewünschten Farbnuancen in ausreichender Intensität ausbilden. Sie müssen ferner ein ausreichendes bis sehr gutes Aufziehvermögen auf menschlichem Haar besitzen, und sie sollen darüber hinaus in toxikologischer und dermatologischer Hinsicht unbedenklich sein.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Es wurde nun gefunden, daß man zu Oxidationshaarfarben, die den gestellten Anforderungen in besonders hohem Maße gerecht werden, gelangt, wenn man als Entwicklerkomponenten und/oder Kupplersubstanzen Bis-Aminopyridine der allgemeinen Formel

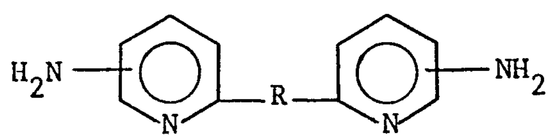

in der R die Gruppen $-HN-CH_2-NH-$, $HN-CH_2-CH_2-NH-$ darstellen kann, sowie deren anorganische oder organische Salze verwendet.

Haarfärbemittel auf Basis von Oxidationsfarbstoffen mit einem Gehalt an Bis-Aminopyridinen der allgemeinen Formel

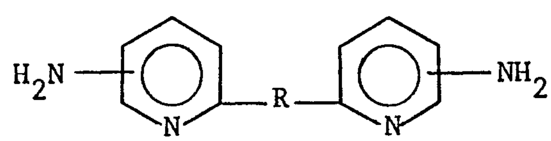

in der R die Gruppen $-HN-CH_2-NH-$, $HN-CH_2-CH_2-NH-$ darstellen kann, sowie deren anorganischen oder organischen Salzen als Entwicklerkomponenten und/oder Kupplersubstanzen und gegebenenfalls den in Oxidationshaarfarben üblichen Kupplersubstanzen, beziehungsweise Entwicklerkomponenten stellen demnach besonders wertvolle Kompositionen auf dem Gebiet der Oxidationshaarfarben dar.

Bei ihrem Einsatz als Entwicklerkomponenten und/oder Kupplersubstanzen liefern die erfindungsgemäßen neuen Verbindungen mit den im allgemeinen für die Oxidationshaarfärbung verwendeten Kupplersubstanzen beziehungsweise Entwicklerkomponenten sowie auch im alleinigen Einsatz die unterschiedlichsten, sehr intensiven Farbnuancen, vor allem interessante Brauntöne und stellen somit eine wesentliche Bereicherung der oxidativen Haarfärbemöglichkeiten dar. Darüber hinaus zeichnen sich die erfindungsgemäßen Bis-Aminopyridine durch sehr gute Echtheitseigenschaften der damit erzielten Färbungen, durch eine gute Löslichkeit in Wasser, eine gute Lagerstabilität und toxikologische sowie dermatologische Unbedenklichkeit aus.

Die erfindungsgemäß als Entwicklerkomponenten und/oder Kupplersubstanzen zu verwendenden

Bis-Aminopyridine können entweder als solche oder in Form ihrer Salze mit anorganischen oder organischen Säuren wie z.B. als Chloride, Sulfate, Phosphate, Acetate, Propionate, Lactate, Citrate eingesetzt werden.

Die Herstellung der erfindungsgemäß zu verwendenden Bis-Aminopyridine erfolgt analog bekannten Verfahren der organischen Chemie durch Umsetzung von 2-Halogen-nitropyridin, insbesondere 2-Chlornitropyridin mit der entsprechenden bivalenten Stickstoffverbindung, (Diamin) und katalytische Reduktion des erhaltenen Reaktions — produktes zu dem entsprechenden Bis-Aminopyridin.

Als erfindungsgemäß einzusetzende Entwicklerkomponenten und/oder Kupplersubstanzen sind z.B. N,N'-Bis-[5-aminopyridyl-(2)]-äthylendiamindihydrochlorid, N,N'-Bis-[3-aminopyridyl-(2)]-äthylendiamintetrahydrochlorid, N,N'-Bis-[5-aminopyridyl-(2)]-diamino-methan-tetrahydrochlorid zu nennen.

Als Beispiele für in den erfindungsgemäßen Haarfärbemitteln einzusetzende Kupplersubstanzen sind α-Naphthol, o-Kresol, m-Kresol, 2,6-Dimethylphenol, 2,5-Dimethylphenol, 3,4-Dimethylphenol, 3,5-Dimethylphenol, Brenzcatechin, Pyrogallol, 1,5-bzw. 1,7-Dihydroxynaphthalin, 5-Amino-2-methylphenol, Hydrochinon, 2,4-Diamino-anisol, m-Toluylendiamin, 4-Aminophenol, Resorcin, Resorcinmonomethyläther, m-Phenylendiamin, 1-Phenyl-3-methyl-pyrazolon-5, 1-Phenyl-3-aminopyrazolon-5, 1-Phenyl-3,5-diketo-pyrazolidin, 1-Methyl-7-dimethyl-amino-4-hydroxy-chinolon-2, 1-Amino-3-acetacetylamino-4-nitrobenzol oder 1-Amino-3-cyanacetylamino-4-nitro-benzol anzuführen.

Als Beispiele für in den erfindungsgemäßen Haarfärbemitteln einzusetzende Entwicklerkomponenten sind primäre aromatische Amine mit einer weiteren in p-Stellung befindlichen funktionellen Gruppe wie p-Toluylendiamin, Monochlor-p-phenylendiamin, p-Dimethylaminoanilin, p-Aminophenol, p-Diaminoanisol bzw. andere Verbindungen der genannten Art, die weiterhin eine oder mehrere funktionelle Gruppen wie OH-Gruppen $NH_2$-Gruppen, NHR-Gruppen, $NR_2$-Gruppen, wobei R einen Alkyl- oder Hydroxyalkylrest mit 1-4 Kohlenstoffatomen darstellt, ferner Diaminopyridinderivate, heterocyclische Hydrazonderivate, 4-Aminopyrazolonderivate wie 4-Amino-1-phenyl-3-carbamoyl-pyrazolon-5, Tetraaminopyrimidine wie 2,4,5,6-Tetramino, 2-Dimethylamino-4,5,6-triamino-, 2,5-Diamino-4,6-bismethylamino-, 2-Piperidino-4,5,6-triamino-, 6-Morpholino-2,4,5-triamino-, 2,4,5-Triamino-6-β-hydroxyäthylaminopyrimidin, N,N-Bis-(β-hydroxyäthyl)-p-phenylendiamin zu nennen.

Wie bereits angedeutet, können die erfindungsgemäß zu verwendenden Bis-Aminopyridine auch als alleinige Farbstoffkomponente in Haarfärbemitteln eingesetzt werden, da sie zur gegenseitigen oxidativen Kupplung befähigt sind.

In den erfindungsgemäßen Haarfärbemitteln werden die Entwicklerkomponenten im allgemeinen in etwa molaren Mengen, bezogen auf die verwendeten Kupplersubstanzen, eingesetzt. Wenn sich auch der molare Einsatz als zweckmäßig erweist, so ist es jedoch nicht nachteilig, wenn die Entwicklerkomponente in einem gewissen Überschuß oder Unterschuß zum Einsatz gelangt.

Es ist ferner nicht erforderlich, daß die Entwicklerkomponente und die Kupplersubstanz einheitliche Produkte darstellen, vielmehr können sowohl die Entwicklerkomponente als auch die Kupplersubstanz Gemische der vorstehend genannten entsprechenden Verbindungen darstellen.

Darüber hinaus können die erfindungsgemäßen Haarfärbemittel andere bekannte und übliche Entwicklerkomponenten bzw. Kupplersubstanzen sowie auch gegebenenfalls übliche direktziehende Farbstoffe im Gemisch enthalten, falls dies zur Erzielung gewisser Farbnuancen erforderlich ist.

Die oxidative Kupplung, d.h. die Entwicklung der Färbung, kann grundsätzlich wie bei anderen Oxidationshaarfarbstoffen auch, durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat, sowie Gemische aus derartigen Wasserstoffperoxidanlagerungsverbindungen mit Kaliumperoxiddisulfat in Betracht.

Die erfindungsgemäß zu verwendenden Bis-Aminopyridine besitzen ferner den Vorteil, daß sie bereits bei oxidativer Kupplung durch Luftsauerstoff voll befriedigende Färbeergebnisse liefern und somit eine Haarschädigung durch das sonst für die oxidative Kupplung eingesetzte Oxidationsmittel vermieden werden kann. Wird jedoch gleichzeitig neben der Färbung ein Aufhelleffekt am Haar gewünscht, so ist die Mitverwendung von Oxidationsmitteln erforderlich.

Die erfindungsgemäßen Haarfärbemittel werden für den Einsatz in entsprechende kosmetische Zubereitungen wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1-3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind z.B. Netz- oder Emulgiermittel vom anionischen oder nichtionogenen Typ wie Alkylbenzolsulfonate, Fettalkoholsulfate, Alkylsulfonate, Fettsäurealkanolamide, Anlagerungsprodukte von Äthylenoxid an Fettalkohole, Verdickungsmittel wie Methylcellulose, Stärke, höhere Fettalkohole, Paraffinöl, Fettsäuren. Ferner Parfümöle und Haarpflegemittel wie Pantothensäure und Cholesterin zu nennen. Die genannten Zusatzstoffe werden dabei in den für diese Zwecke üblichen Mengen eingesetzt, wie z.B. Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemäßen Haarfärbemittel kann, unabhängig davon, ob es sich um

eine Lösung, eine Emulsion, eine Creme oder ein Gel handelt, im schwach sauren, neutralen oder insbesondere alkalischen Milieu bei einem pH-Wert von 8 bis 10 erfolgen. Die Anwendungstemperaturen bewegen sich dabei im Bereich von 15 bis 40 °C. Nach einer Einwirkungsdauer von ca. 30 Minuten wird das Haarfärbemittel vom zu färbenden Haar durch Spülen entfernt. Hiernach wird das Haar mit einem milden Shampoo nachgewaschen und getrocknet.

Die mit den erfindungsgemäßen Haarfärbemitteln erzielbaren Farbtöne zeigen unter Einsatz unterschiedlicher Entwickler- und Kupplerkomponenten eine außerordentliche Variationsmöglichkeit, die von hellbraun bis dunkelbraun und rotbraun sowie khakifarben und dunkelgrün reicht. Die erzielten Färbungen haben gute Licht-, Wasch- und Reibechtheitseigenschaften und lassen sich leicht mit Reduktionsmitteln wieder abziehen.

Die nachfolgenden Beispiele sollen den Erfindungsgegenstand näher erläutern, ohne ihn jedoch hierauf zu beschränken.

## Beispiele

Zunächst wird die Herstellung einiger neuer erfindungsgemäßer Bis-Aminopyridine beschrieben.

### Beispiel 1 Produkt A

N,N'-Bis-[5-aminopyridyl-(2)-]-äthylendiamindihydrochlorid

Zu einer äthanolischen Lösung von 15,9 g 2-Chlor-5-nitropyridin wurden 3 g Äthylendiamin zugetropft. Das Reaktionsgemisch wurde 1 Stunde lang unter Rückfluß gekocht und nach dem Abkühlen wurde das gelbe Reaktionsprodukt, N,N'-Bis-[5-nitropyridyl-(2)-]-äthylendiamin abgesaugt. Die Verbindung hat einen Schmelzpunkt von 248-255 °C.

5 g des erhaltenen Zwischenproduktes in 50 ml Äthanol wurden katalytisch (10 % Palladium auf Kohle) reduziert. Nach beendeter Wasserstoffaufnahme wurde vom Katalysator abfiltriert und die Lösung mit verdünnter Salzsäure angesäuert. Beim Einengen zur Trockne wurden 5 g des N,N'-Bis-[5-aminopyridyl-(2)]-äthylendiamindihydrochlorids als braunes Pulver erhalten. Das Massenspektrum zeigte die Molekülmasse 268 (ber. 268).

### Beispiel 2 Produkt B

N,N'-Bis-[3-aminopyridyl-(2)]-äthylendiamintetrahydrochlorid

Entsprechend den Angaben in Beispiel 1 wurden 15,9 g 2-Chlor-3-nitropyridin in äthanolischer Lösung mit 2,4 g Äthylendiamin zu N,N'-Bis-[3-nitropyridyl-(2)]-äthylendiamin umgesetzt. Es wurden 10,3 g eines gelben Pulvers vom Schmelzpunkt 125 °C erhalten.

Analog zu den Angaben in Beispiel 1 wurden 5 g des erhaltenen Zwischenproduktes in Äthanol katalytisch reduziert und angesäuert. Es wurden 4,5 g N,N'-Bis-[3-aminopyridyl-(2)]-äthylendiamintetrahydrochlorid als braunes Pulver erhalten.

### Beispiel 3 Produkt C

N,N'-Bis-[5-aminopyridyl-(2)]-diaminomethan-tetrahydrochlorid

5 g N,N'-Bis-[5-nitropyridyl-(2)]-diaminomethan, das gemäß den Angaben von A.E. Tschitschibabin und J.L. Kunjanz in Chem. Ber. 62 (1929) 3 052 hergestellt worden war, wurden in 100 ml Äthanol in Gegenwart von 1 g Palladium auf Kohle katalytisch reduziert. Nach beendeter Wasserstoffaufnahme wurde in 150 ml verdünnte Salzsäure filtriert und eingeengt. Es wurden 2,5 g eines orangefarbenen Pulvers, das bei 240-244 °C unter Zersetzung schmolz, erhalten.

Die erfindungsgemäßen Haarfärbemittel wurden in Form einer Cremeemulsion eingesetzt. Dabei wurden in eine Emulsion aus

10 Gew.-Teilen Fettalkoholen der Kettenlänge $C_{12}$-$C_{18}$
10 Gew.-Teilen Fettalkoholsulfat (Natriumsalz) Kettenlänge $C_{12}$-$C_{18}$
75 Gew.-Teilen Wasser

jeweils 0,01 Mol der in der nachstehenden Tabelle aufgeführten Entwicklerkomponenten und Kupplersubstanzen eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde entweder mit Luftsauerstoff oder mit 1 %iger Wasserstoffperoxidlösung als Oxidationsmittel durchgeführt, wobei zu 100 Gewichtsteilen der Emulsion 10 Gewichtsteile Wasserstoffperoxidlösung gegeben wurden.

Die jeweilige Färbecreme mit oder ohne zusätzlichem Oxidationsmittel wurde auf zu 90 % ergrautes, nicht besonders vorbehandeltes Menschenhaar aufgetragen und dort 30 Minuten belassen. Nach

Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

In Kombination mit den erfindungsgemäß zu verwendenden Bis-Aminopyridinen wurden dabei als weitere Farbstoffkomponenten folgende Verbindungen eingesetzt :

Produkt 1 α-Naphthol.
Produkt 2 Resorcin.
Produkt 3 p-Toluylendiamin.
Produkt 4 2,4,5,6-Tetraaminopyrimidin.
Produkt 5 2,4-Diaminoanisol.
Produkt 6 Resorcinmonomethyläther.

TABELLE 1

| Beispiel | Entwickler | Kuppler | Erhaltener Farbton | |
|---|---|---|---|---|
| | | | bei Luft-oxidation | mit 1 % $H_2O_2$ |
| 4 | Produkt B | Produkt 4 | braungrau | braun |
| 5 | Produkt B | Produkt 3 | braungrau | braungrau |
| 6 | Produkt B | Produkt 1 | mattgrün | türkis |
| 7 | Produkt B | Produkt 5 | mattgrün | dunkelgrün |
| 8 | Produkt B | Produkt 2 | braun | braun |
| 9 | Produkt A | Produkt 4 | dunkelbraun | dunkelbraun |
| 10 | Produkt A | Produkt 3 | braun | dunkelbraun |
| 11 | Produkt A | Produkt 6 | rotbraun | rotbraun |
| 12 | Produkt A | Produkt 5 | grün | dunkelgrün |
| 13 | Produkt 4 | Produkt C | braun | braun |
| 14 | Produkt 3 | Produkt C | gelbbraun | dunkelbraun |
| 15 | Produkt C | Produkt 1 | khakifarben | gelbbraun |
| 16 | Produkt C | Produkt 2 | khakifarben | gelbbraun |

**Ansprüche**

1. Bis-Aminopyridine der allgemeinen Formel

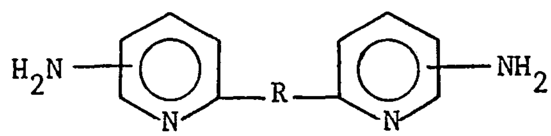

in der R die Gruppen —HN—$CH_2$—NH, HN—$CH_2$—$CH_2$—NH— darstellt, sowie deren Salze mit anorganischen oder organischen Säuren.

2. N,N'-Bis-[5-aminopyridyl-(2)]-ethylendiamin, N,N'-Bis-[3-amino-pyridyl-(2)]-ethylendiamin, sowie N,N'-Bis-[5-aminopyridyl-(2)]-diaminomethan und deren Hydrochloride nach Anspruch 1.

3. Verfahren zur Herstellung der Bis-Aminopyridine nach Anspruch 1 und 2 durch Umsetzung von entsprechenden 2-Halogen-nitropyridinen, insbesondere 2-Chlor-nitro-pyridinen mit der entsprechenden bivalenden Stickstoffverbindung, (Diamin) und katalytische Reduktion des erhaltenen Reaktionsproduktes.

4. Verwendung von Bis-Aminopyridinen sowie deren Salzen mit anorganischen oder organischen Säuren nach Anspruch 1 und 2 als Entwicklerkomponenten und/oder Kupplersubstanzen in Oxidationshaarfarbstoffen.

5. Haarfärbemittel auf Basis von Oxidationsfarbstoffen mit einem Gehalt an Bis-Aminopyridinen sowie deren Salzen mit anorganischen oder organischen Säuren nach Anspruch 1 und 2 als Entwicklerkomponenten und/oder Kupplersubstanzen sowie gegebenenfalls den in Oxidationshaarfarben üblichen Entwicklerkomponenten oder Kupplersubstanzen.

6. Haarfärbemittel nach Anspruch 5, gekennzeichnet durch einen Gehalt an Entwickler-Kuppler-Kombinationen von 0,2 bis 5 Gewichtsprozent, vorzugsweise 1 bis 3 Gewichtsprozent.

**Claims**

1. Bis-aminopyridines corresponding to the following general formula

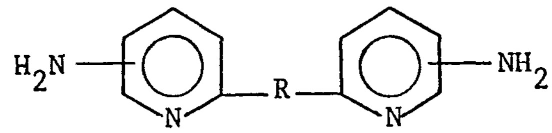

in which R represents the groups —HN—$CH_2$—NH—, HN—$CH_2$—$CH_2$—NH—, and their salts with inorganic or organic acids.

2. N,N'-bis-[5-amino-2-pyridyl]-ethylene diamine, N,N'-bis-[3-amino-2-pyridyl]-ethylene diamine and N,N'-bis-[5-amino-2-pyridyl]-diaminomethane and their hydrochlorides as claimed in Claim 1.

3. A process for producing the bis-aminopyridines claimed in Claims 1 and 2 by reacting corresponding 2-halogen nitropyridines, particularly 2-chloronitropyridines, with the corresponding divalent nitrogen compound (diamine) and catalytically reducing the reaction product obtained.

4. The use of the bis-aminopyridines and their salts with inorganic or organic acids claimed in Claims 1 and 2 as developer components and/or couplers in oxidative hair dyes.

5. Hair dyes based on oxidative dyes containing bis-aminopyridines and their salts with inorganic or organic acids as claimed in Claims 1 and 2 as developer components and/or couplers and, optionally, the developer components or couplers normally used in oxidative hair dyes.

6. Hair dyes as claimed in Claim 5, characterised by a content of from 0.2 to 5 % by weight and preferably from 1 to 3 % by weight of developer/coupler combinations.

**Revendications**

1. Bis-aminopyridines de formule générale

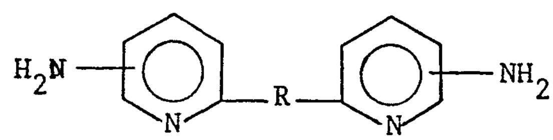

dans laquelle R représente les groupes —HN—$CH_2$—NH, —HN—$CH_2$—$CH_2$—NH—, ainsi que leurs sels avec des acides minéraux ou organiques.

2. N,N'-bis-[5-aminopyridyl-(2)]-éthylène diamine, N,N'-bis-[3-aminopyridyl-(2)]-éthylène diamine de même que N,N'-bis-[5-aminopyridyl-(2)]-diaminométhane et leurs chlorhydrates selon la revendication 1.

3. Procédé de préparation des bis-aminopyridines selon les revendications 1 et 2 par réaction des 2-halogéno-nitropyridines correspondantes, en particulier des 2-chloronitro-pyridines, avec le composé azoté bivalent correspondant (diamine) et réduction catalytique du produit de réaction obtenu.

4. Utilisation des bis-aminopyridines de même que de leurs sels avec des acides minéraux ou organiques selon les revendications 1 et 2 comme composants de développement et/ou comme substances de couplage dans des colorants d'oxydation pour cheveux.

5. Agent de teinture des cheveux à base de colorants d'oxydation ayant une teneur en bis-aminopyridines de même que de leurs sels avec des acides minéraux ou organiques selon les revendications 1 et 2 comme composants de développement et/ou comme substances de couplage, de même qu'éventuellement en composants de développement ou substances de couplage qui sont habituels dans les colorants d'oxydation pour cheveux.

6. Agent de teinture des cheveux selon la revendication 5, caractérisé par une teneur en des combinaisons agent de développement-coupleur de 0,2 à 5 % en poids, de préférence de 1 à 3 % en poids.